# FASCICULE DE BREVET EUROPEEN

(11) **EP 2 172 186 B1**
(45) Date de publication et mention de la délivrance du brevet: **30.11.2016**
(21) Numéro de dépôt: 09368034.6
(22) Date de dépôt: 29.09.2009
(51) Int. Cl.: A61K 8/58, A61K 8/73, A61Q 19/08

(54) **Complexe associant un dérivé organique du silicium avec des fragments calibrés d'acide hyaluronique, à action préventive et réparatrice des dégradations cutanées**
Komplex bestehend aus einem organischen Silizium-Derivat und kalibriert Hyaluronsäure-Fragmenten, zur Vorbeugung und Behandlung des Haut-Abbaus
Complex comprising an organic silicon derivative and calibrated hyaluronic acid fragments, for preventing and restoring cutaneous degradation

(30) Priorité: 03.10.2008 FR 0805479
(43) Date de publication de la demande: 07.04.2010
(73) Titulaire: Exsymol S.A.M., 98000 Monaco (MC)
(72) Inventeur: Seguin, Marie-Christine, 98000 Monaco (MC)
(74) Mandataire: Macquet, Christophe

(56) Documents cités:
- EP-A- 0 289 366
- FR-A- 2 865 651
- FR-A- 2 865 935
- "Very low molecular weight hyaluronic acid for antiaging and antiwrinkle treatment" , [Online] 29 janvier 2008 (2008-01-29), XP002535445 Extrait de l'Internet: URL:http://web.archive.org/web/20080129193 022/biopolymer.novozymes.com/downloads/> [extrait le 2009-06-08]

## Description

L'invention concerne un complexe associant un dérivé organique du silicium avec un ou plusieurs fragments calibrés d'acide hyaluronique, et son utilisation dans la prévention ou la réparation des dégradations cutanées.

Polysaccharide linéaire constitué d'unités répétitives d'acide D-glucuronique et de N-acétyl-D-glucosamine, l'acide hyaluronique est retrouvé dans la matrice extra-cellulaire de nombreux tissus conjonctifs (peau, tendons, muscles, etc.), plus exactement sous la forme d'un polyanion appelé "hyaluronane".
Dans la peau par exemple, l'acide hyaluronique ("HA" ci-après) est l'un des composants majeurs. Il est en effet retrouvé en quantité importante dans les matrices extra-cellulaires dermiques et épidermiques. Ses propriétés hydrophiles et visco-élastiques en font un acteur essentiel dans le maintien de l'hydratation, du volume et de la cohésion cutanée.

Le HA synthétisé par les fibroblastes et les kératinocytes ("HA natif" ci-après) existe majoritairement sous forme de polymères de très haut poids moléculaire (> à 2.000 kDa), pouvant même atteindre 4.000 kDa avec une chaîne de 10.000 disaccharides (Hascall V.C. et al., GlycoForum/Hyaluronan Today (1997), chapitre 1).
Cependant, le HA natif est soumis dans la peau à un ensemble de réactions de dégradation physiologique, notamment enzymatiques, appelé catabolisme, qui vise à le réduire en de plus petits fragments ("HA fragmenté" ci-après). Depuis plusieurs années, des équipes de recherche ont porté leur attention sur le catabolisme de HA et ses conséquences dans l'organisme. Et la conclusion de tous ces travaux est en général unanime, à savoir que le HA fragmenté possède un comportement différent du HA natif, et que selon la taille des fragments, c'est-à-dire leur poids moléculaire, on observe des effets biologiques différents, voire même opposés (Noble P.W., Matrix Biol. (2002), vol. 21, pp. 25-29 ; Asari A., GlycoForum/Hyaluronan Today (2005), chapitre 29 et références citées).

Il est ainsi rapporté, pour des fragments de HA dits de haut poids moléculaire, un effet sur la régénération et la cicatrisation, un rôle important comme régulateur du processus inflammatoire, mais aussi un effet immunosuppresseur. En revanche, des oligomères dits de faible poids sont présentés comme des entités capables d'activer les cellules immunes et de délivrer des signaux endogènes vis-à-vis de stress, mais aussi d'être de puissants inducteurs de l'inflammation et de l'angiogénèse (Stern R., Clin. Dermatol. (2008), vol. 26, pp. 106-122 ; Krasinski R. et al., Postepy Hig. Med. Dosw (2007), vol. 61, pp. 683-689).

Concernant la peau plus précisément, il est rapporté une stimulation de la prolifération de kératinocytes en culture, pour des fragments annoncés de "taille intermédiaire" avec un poids moléculaire compris entre 50 et 400 kDa (Kaya G. et al., PLoS Medicine (2006), vol. 3, pp. 2291-2303). Ces effets sont aussi observés après application topique d'une préparation à base de fragments de HA de poids moléculaire compris entre 50 et 750 kDa (qualifiés de "bas poids moléculaire" dans la partie descriptive de la demande FR 2 865 651). Cette avantageuse propriété, notamment synonyme d'une meilleure fonction barrière de la peau avec une épaisseur de l'épiderme augmentée, ne serait cependant plus observée avec de petits fragments de HA inférieurs à 50 kDa (Kaya G. et al., PLoS Medicine (2006), vol. 3, pp. 2291-2303).

Bien qu'il ait été longtemps considéré qu'un passage transcutané de HA, pour un bénéfice au-delà de la simple surface de la peau et de la couche cornée, ne pouvait être envisagé que pour des fragments de HA de très faible poids moléculaire (< à 50 kDa, Tammi R. et al., J. Invest. Dermatol. (1991), vol. 97, pp. 126-130), il est désormais fait état d'une capacité de diffusion dans l'épiderme pour des fragments de HA de taille nettement plus élevée, à savoir pour des fragments de 360 à 400 kDa (Brown T.J. et al., J. Invest. Dermatol. (1999), vol. 113, pp. 740-746) ou encore pour les fragments intermédiaires susmentionnés (50-400 kDa). Cette diffusion irait même jusqu'à pouvoir atteindre le derme avec la préparation topique également évoquée plus haut à base de HA (50-750 kDa).

Dans le domaine cosmétique, et notamment celui de la lutte contre le vieillissement cutané, il peut apparaître opportun d'apporter topiquement à la peau de l'acide hyaluronique fragmenté de taille appropriée, et ce, afin de s'opposer aux effets du vieillissement ou de facteurs extrinsèques (radicaux libres, rayonnements ultra-violets, pollution, etc). Il est en effet admis que le vieillissement ou les rayonnements ultra-violets influencent le catabolisme de HA et la libération de fragments, et peuvent résulter une production intracellulaire plus faible et une distribution plus inégale (Meyer et al., J. Invest. Dermatol. (1994), vol. 3, pp. 385-389). Cependant, une application cosmétique ne saurait s'accommoder d'effets secondaires indésirables, tels l'effet pro-inflammatoire annoncé ci-dessus pour de petits fragments d'acide hyaluronique.

Aussi, compte tenu de ce qui précède conjugué au besoin de produits nouveaux avec le confort d'une application topique, le problème technique que se propose de résoudre l'invention est de développer un nouvel ingrédient actif à base de fragments d'acide hyaluronique, avec le double objectif :
- potentialiser/amplifier les effets biologiques reconnus aux fragments d'acide hyaluronique dits de faible ou moyen poids moléculaire,
- éviter l'effet adverse pro-inflammatoire des petits fragments produits par l'action catabolique des tissus cutanés.

Le choix de la demanderesse s'est arrêté sur une entité moléculaire, sous forme de complexe, associant un dérivé organique du silicium avec des fragments calibrés d'acide hyaluronique de poids moléculaire compris entre 150 et 750 kDa, pour les deux raisons suivantes.

En premier lieu, ledit complexe a constitué une réponse avantageuse aux objectifs énoncés précédemment, illustré par :
- la mise en évidence, sur un modèle d'épiderme humain reconstruit, d'un effet cytostimulant largement supérieur en comparaison à celui observé pour les mêmes fragments d'acide hyaluronique mais non associés [cf test 1 ci-après], suggérant ainsi un effet de synergie entre les parties siliciées et hyaluronique du complexe,
- une véritable tolérance accompagnant ce renouvellement cellulaire puisqu'il n'est pas observé d'induction significative de cytokines pro-inflammatoires par rapport au contrôle utilisé [cf test 2 ci-après]. Cette induction se trouve même être réduite pour l'une des cytokines testées.

En second lieu, à l'étonnement de la demanderesse, il a été constaté, lors d'une étude *in vitro* sur la dégradation cinétique de fragments de HA à l'aide de hyaluronidases, une moindre sensibilité et accessibilité de ces fragments de HA à ces enzymes dès lors que ces fragments se trouvent être associés sous forme de complexe à un dérivé organique de silicium [cf test 3 ci-après]. En effet, en comparaison à du HA fragmenté de même poids mais non associé, une électrophorèse sur gel d'agarose a mis en évidence la présence d'une plus importante distribution de fragments de HA de poids moléculaire élevé. Un tel comportement est appréciable dans le contexte de l'invention, car il est la preuve d'un catabolisme ralenti, suggérant aussi un rôle protecteur du dérivé organique de silicium sur les fragments de HA sous forme de complexe.

Dans l'état de la technique, il est certes à relever un produit à visée cosmétique de formule RₙSi(OR')ₘ(OR")ₚ combinant un composé organo-silicié avec un composé biologiquement actif (R'), notamment l'acide hyaluronique cité à titre d'exemple non limitatif, et une molécule dermatophile (R") dont le rôle est d'empêcher le passage du silicium organique dans les tissus sous-jacents de la peau (brevet EP 0 289 366). Les radicaux R et R' sont cependant de portée excessivement large, choisis parmi de simples composés organiques porteurs d'une ou de plusieurs fonctions alcool, phénol, acide, amine ou aminoacide. En outre dans ce document, et même si l'acide hyaluronique est listé, il n'y est pas fait mention de l'utilisation de fragments de HA de taille calibrée, ni d'une efficacité particulière sur le renouvellement cellulaire épidermique tel qu'il est observé avec la présente invention. Les mêmes remarques s'appliquent aux composés organo-siliciés sous forme solide dans la demande européenne EP 0 867 445, ainsi que pour le produit hydratant objet de la demande FR 2 561 915. Dans cette référence française, il est proposé l'association d'un silanol et d'une substance hydrosoluble clairement à l'état de polymère, telle des protéines, des mucopolysaccharides, notamment l'acide hyaluronique, ou encore des dérivés de la cellulose, et pour son seul effet de conservation d'humidité et d'absence de sensation poisseuse sur la peau.

Le document FR2865935 divulgue des compositions comprenant de l'hyaluronate de monométhylsilanetriol ou de l'hyaluronate de diméthylsilanetriol dans le traitement de tissus conjonctifs lésés, tels que le derme de la peau, notamment le traitement d'altérations dermiques induites par le vieillissement; sans toutefois divulguer la taille des fragments d'acide hyaluronique utilisés.

Ainsi, selon un premier aspect, l'invention a pour objet un complexe à base d'un dérivé organique de silicium caractérisé en ce que ledit dérivé est associé, par des liaisons faibles, à un ou plusieurs fragments calibrés d'acide hyaluronique de poids moléculaire compris entre 150 et 750 kDa.

Dans un mode de réalisation préféré de l'invention, le poids moléculaire de ces fragments est compris entre 150 et 600 kDa, de manière encore plus préférée entre 250 et 600 kDa.

Par "dérivé organique de silicium", il faut comprendre un composé défini par la formule générale (I) suivante :

R_{X}Si(OH)_{4-X} (I)

dans laquelle :
R est un (C₁-C₄)alkyle,
x = 1 et 2.

Les dérivés organiques de silicium selon l'invention se présentent sous la forme de monomères, dimères ou trimères, essentiellement des monomères ou dimères, ou d'un mélange de monomères, dimères et trimères, essentiellement d'un mélange de monomères et dimères, avec un état en solution pouvant être représenté par la formule (II) suivante :

[(T0)ᵣ + (T1)ₛ + (T2) t] (II)

dans laquelle :
T0, T1 et T2 ont respectivement les formules : 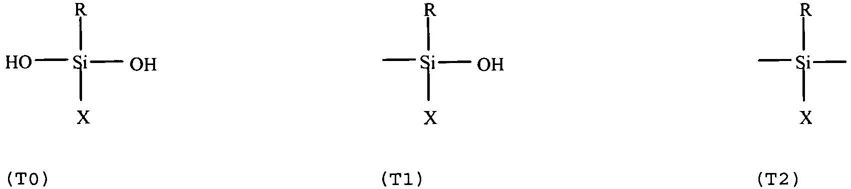 où :
   R est tel que défini ci-dessus pour les composés (I),
   X représente un groupe hydroxyle ou un radical R tel que défini ci-dessus, et
   r, s et t sont tels que 1 ≤ r+s+t ≤ 3.

Dans les formules (I) et (II) ci-dessus, on entend par "groupe alkyle en C₁-C₄" une chaîne hydrocarbonée ayant de 1 à 4 atomes de carbone, linéaire, ramifiée, ou bien encore cyclique. Un tel groupe est notamment un groupe méthyle, éthyle, propyle, butyle, 1-méthyléthyle, 1-méthylpropyle, 2-méthylpropyle, 1,1-diméthyléthyle et cyclopropylméthyle.

Par "fragments d'acide hyaluronique", il faut comprendre des fragments issus de HA natif en tant que tel ou de l'un quelconque de ses sels, particulièrement des fragments de hyaluronate de sodium.

Suivant un autre mode de réalisation avantageux de l'invention, le dérivé organique de silicium de formule (I) est choisi parmi le monométhylsilanetriol ou le diméthylsilanediol. Il est particulièrement choisi le monométhylsilanetriol, et plus particulièrement encore le monométhylsilanetriol associé à un ou plusieurs fragments d'acide hyaluronique de poids moléculaire compris entre 150 et 600 kDa (complexe "MSH" ci-après) ou entre 250 et 600 kDa.

Selon un autre aspect, la présente invention s'étend à une composition cosmétique d'application topique comprenant, en association avec tout excipient physiologiquement compatible avec la peau, à titre d'ingrédient actif principal, le complexe tel que défini précédemment, particulièrement à base du monométhylsilanetriol, et plus particulièrement encore à base du monométhylsilanetriol associé à un ou plusieurs fragments d'acide hyaluronique de poids moléculaire compris entre 150 et 600 kDa ou entre 250 et 600 kDa, ladite composition étant destinée à prévenir ou à réparer les dégradations cutanées liées au vieillissement pour lesquelles il est nécessaire de relancer l'activité cellulaire épidermique tout en limitant l'action catabolique du tissu cutané.

Avantageusement, la quantité du complexe de formule (I) dans la composition ci-dessus est comprise entre 1 et 10 % en poids par rapport au poids total de la composition, de préférence entre 2 et 5 % en poids.

On peut citer, à titre d'exemple d'excipient physiologiquement compatible avec la peau, un tensioactif, un conservateur, un corps gras, un colorant, un émulsionnant, un gélifiant, un émolliant, un humectant, un pigment, un antioxydant ou tout autre adjuvant habituellement utilisé en cosmétique.

Les compositions selon l'invention sont adaptées à une administration par voie topique cutanée, présentées sous toutes les formes normalement utilisées pour une telle administration. De manière avantageuse, elles sont formulées sous forme, par exemple, de crème, lait, gel, lotion, émulsion, etc.

Selon un autre aspect, l'invention a également pour objet l'utilisation d'un complexe tel que défini précédemment comme agent cytostimulant destiné à relancer l'activité cellulaire épidermique (kératinocytes) tout en limitant l'action catabolique du tissu cutané. Il est particulièrement utilisé le monométhylsilanetriol, et plus particulièrement encore le monométhylsilanetriol associé à un ou plusieurs fragments d'acide hyaluronique de poids moléculaire compris entre 150 et 600 kDa ou entre 250 et 600 kDa.

Enfin, selon un dernier aspect, la présente invention concerne un procédé de soin cosmétique destiné à prévenir ou à réparer les dégradations cutanées liées au vieillissement et pour lesquelles il est nécessaire de relancer l'activité cellulaire épidermique, procédé mis en oeuvre en appliquant sur la peau une composition telle que définie précédemment, particulièrement à base du monométhylsilanetriol et plus particulièrement encore à base du monométhylsilanetriol associé à un ou plusieurs fragments d'acide hyaluronique de poids moléculaire compris entre 150 et 600 kDa ou entre 250 et 600 kDa.

A titre d'illustration, il est mentionné ci-après deux exemples de formulation de composition cosmétique selon l'invention, successivement avec le monométhylsilanetriol associé à des fragments d'acide hyaluronique de poids moléculaire compris entre 150 et 600 kDa (formule A), et avec le diméthylsilanediol associé à des fragments d'acide hyaluronique de poids moléculaire compris entre 250 et 600 kDa (formule B) :

**Formule A (crème)**

| | |
|---|---|
| Complexe MSH (monométhylsilanetriol/fragments HA 150-600 kDa) | 5 % |
| Polyisobutène hydrogéné | 7 % |
| Myristate d'isobutyle | 3 % |
| Palmitate de cétyle | 7 % |
| Monostéarate d'éthylène glycol | 5 % |
| Laurate sorbitan | 2 % |
| Polysorbate 20 | 2 % |
| Carbomer (copolymère d'acrylate/acrylamide & huile minérale) | 0,3 % |
| Phénoxyéthanol | 0,5 % |
| Eau | qsp 100 % |

**Formule B (gel)**

| | |
|---|---|
| Complexe (diméthylsilanediol/fragments HA 250-600 kDa) | 5 % |
| Carbomer (copolymère d'acrylate/acrylamide & huile minérale) | 1,5 % |
| Benzoate de sodium | 0,2 % |
| Acide sorbique | 1 % |
| 1,3-butanediol | 10 % |
| Glycérine | 5 % |
| Soude | 0,13 % |
| Phénoxyéthanol | 0,9 % |
| Eau | qsp 100 % |

L'invention est illustrée ci-après, à titre purement indicatif, par les tests suivants évoqués plus haut dans la description (tests 1 à 3), et une étude comparative (test 4). Dans les tests 1, 2 et 4, les études expérimentales ont été menées sur un modèle d'épiderme humain reconstruit (EHR, fournisseur : SkinEthic^{®}) dont l'intérêt est sa forte homologie avec l'épiderme humain.

### Test 1 : mise en évidence de l'effet cytostimulant du complexe MSH sur des épidermes reconstruits d'origine humaine

La mise en culture des EHR a consisté à les placer dans un milieu de croissance "MCDB 153" (fournisseur : SkinEthic^{®}) contenant 5mg/mL d'insuline, 1,5 mM de CaCl₂ et 25 mg/mL de gentamycine, pendant 24 heures à 37°C et 5% CO₂.

L'observation de la prolifération cellulaire est réalisée par la technique de l'immunomarquage (Gerdes et al., Int. J. Cancer (1983), vol. 31, pp. 13-20) en utilisant le marqueur de prolifération cellulaire "Ki67", pour les trois configurations suivantes (après un dépôt biquotidien de 100 µl sur les EHR) :
- cas 1 : solution tampon de PBS (contrôle)
- cas 2 : fragments de HA 150-600 kDa (conc. 2 et 5 %)
- cas 3 : complexe MSH (conc. 2 et 5 %)

Les résultats obtenus sont rassemblés dans le tableau 1 ci-après :

**Tableau 1**

| | **Concentration (%)** | **% cellules exprimant Ki67 / nombre de cellules totales** (% par rapport au contrôle) |
|---|---|---|
| cas 1 (contrôle) | - | - |
| cas 2 (HAF 150-600 kDa) | 2,5 | 0 |
| cas 2 (HAF 150-600 kDa) | 5 | + 28 (± 0,6) |
| cas 3 (complexe MSH) | 2,5 | + 14 (± 1,0) |
| cas 3 (complexe MSH) | 5 | + 63 (± 0,5) |

Les résultats, rassemblant les valeurs obtenues à partir de trois expérimentations indépendantes, soulignent une cytostimulation potentialisée pour le complexe MSH selon l'invention, car très supérieure à celle observée pour le HA fragmenté non associé.

### Test 2 : mise en évidence de l'absence d'induction de réponse pro-inflammatoire pour le complexe MSH

La mise en culture des EHR est identique à celle du test 1 ci-avant. Le dosage des interleukines pro-inflammatoires IL1a et IL8 est réalisé sur un surnageant issu du milieu de culture après 24 heures de traitement, à l'aide de kits de dosage ELISA (R&D System Quantikine Immunoassay, D800C, DTA00C et DLA50), et pour des configurations identiques à celles du test 1 ci-dessus.
Les résultats obtenus sont rassemblés dans le tableau 2 ci-après :

**Tableau 2**

| | **Concentration (%)** | **Quantité IL1α (pg/ml)** | **Quantité IL8 (pg/ml)** |
|---|---|---|---|
| cas 1 (contrôle) | - | 1,8 (± 0,1) | 38,8 (± 11,5) |
| cas 2 (HAF 150-600 kDa) | 2,5 | 1,4 (± 0) | 40,2 (± 4,9) |
| cas 2 (HAF 150-600 kDa) | 5 | 0,6 (± 0,3) | 31,0 (± 0,6) |
| cas 3 (complexe MSH) | 2,5 | 3,5 (± 1,8) | 20,1 (± 7,2) |
| cas 3 (complexe MSH) | 5 | 3,4 (± 0,8) | 34,8 (± 13,7) |

Nulle induction significative de cytokines pro-inflammatoires n'est observée pour le complexe MSH selon l'invention. Il est même observé, tant par rapport au contrôle que celui du HA fragmenté non associé, une réduction du taux des interleukines de type IL8.

### Test 3 : mise en évidence d'un catabolisme ralenti sur gel d'agarose pour le complexe MSH

L'étude expérimentale a consisté à déterminer, par électrophorèse sur gel d'agarose, la cinétique de dégradation du complexe MSH selon l'invention soumis *in vitro* à des hyaluronidases d'origine bovine. 10µl de hyaluronidases à 1 mg/mL sont mélangés à 50µl du complexe dans une solution tampon de PBS pendant 30 minutes à 37°C.

Successivement à 5, 15 et 30 minutes, il est prélevé 10µl de mélange réactionnel qui sont ensuite chauffés à 95°C afin de stopper la réaction enzymatique. Ces 10µl sont alors déposés sur un gel d'agarose et un colorant approprié (Stains all^{®}) est utilisé pour la révélation de l'électrophorèse.

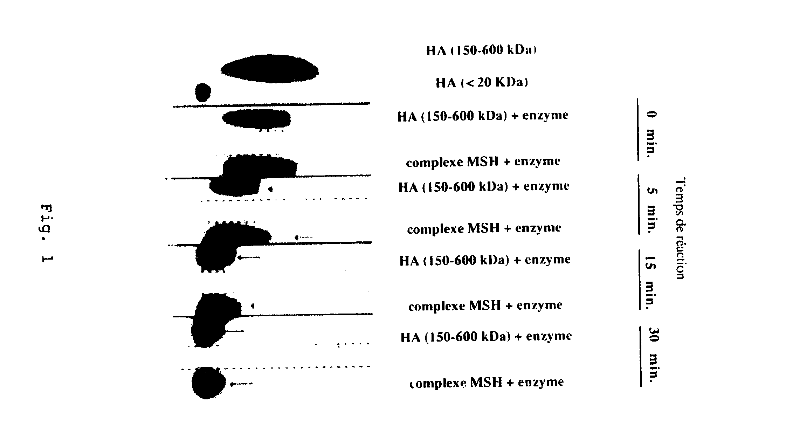

Comme le montre le gel d'agarose représenté à la figure 1 ci-après, l'électrophorèse révèle, pour les trois temps de prélèvement et de réaction, une plus importante distribution de HA avec un poids moléculaire élevé pour le complexe MSH selon l'invention, comparé au HA fragmenté non associé.

### Test 4 : comparaison des effets cytostimulants du complexe MSH et d'un hyaluronate de diméthylsilanediol de haut poids moléculaire

Identiquement au test 1 ci-avant, la prolifération cellulaire de kératinocytes est observée sur des épidermes humains reconstruits EHR (par la technique de l'immunomarquage) pour :
- le complexe MSH selon l'invention (à base de fragments HA 150-600 kDa)
- le hyaluronate de diméthylsilanediol, commercialisé par la demanderesse sous le nom de marque "D.S.H. CN^{®}". D.S.H. CN^{®} est un produit organo-silicié issu de la combinaison du diméthylsilanediol avec l'acide hyaluronique de haut poids moléculaire (fragments HA 1500 2200 kDa)

Les résultats obtenus sont rassemblés dans le tableau 4 ci-après, toujours par rapport à une solution tampon de PBS (contrôle) :

**Tableau 4**

| | **Concentration (%)** | **% cellules exprimant Ki67 / nombre de cellules totales** (% par rapport au contrôle) |
|---|---|---|
| contrôle | - | - |
| D.S.H. CN^{®} | 5 | 0 (±2,2) |
| complexe MSH | 5 | + 69,5 (± 3,1) |

## Revendications

1. Complexe à base d'un dérivé organique de silicium **caractérisé en ce que** ledit dérivé est associé, par des liaisons faibles, à un ou plusieurs fragments calibrés d'acide hyaluronique de poids moléculaire compris entre 150 et 750 kDa, ledit dérivé étant de formule générale (I) :
RₓSi(OH)₄₋ₓ (I)
dans laquelle :
R est un (C₁-C₄)alkyle,
x = 1 et 2.

2. Complexe selon la revendication 1, **caractérisé en ce que** le poids moléculaire desdits fragments est compris entre 150 et 600 kDa.

3. Complexe selon l'une des revendications 1 et 2, **caractérisé en ce que** le poids moléculaire desdits fragments est compris entre 250 et 600 kDa.

4. Complexe selon l'une quelconque des revendications 1 à 3, **caractérisé en ce que** ledit dérivé organique de silicium de formule (I) est choisi parmi le monométhylsilanetriol ou le diméthylsilanediol.

5. Complexe selon la revendication 4, **caractérisé en ce que** le dérivé organique de silicium est le monométhylsilanetriol.

6. Complexe selon l'une quelconque des revendications 1 à 5, **caractérisé en ce qu'**il s'agit du monométhylsilanetriol associé à un ou plusieurs fragments d'acide hyaluronique de poids moléculaire compris entre 150 et 600 kDa ou entre 250 et 600 kDa.

7. Composition cosmétique d'application topique destinée à prévenir ou à réparer les dégradations cutanées liées au vieillissement **caractérisée en ce qu'**elle comprend, en association avec tout excipient physiologiquement compatible avec la peau, à titre d'ingrédient actif principal, un complexe selon l'une quelconque des revendications 1 à 6.

8. Composition selon la revendication 7, **caractérisée en ce que** la quantité du complexe de formule (I) est comprise entre 1 et 10 % en poids par rapport au poids total de la composition, de préférence entre 2 et 5 % en poids.

9. Composition selon l'une quelconque des revendications 7 et 8, **caractérisée en ce qu'**elle est formulée sous forme de crème, lait, gel, lotion, émulsion.

10. Composition selon l'une quelconque des revendications 7 à 9, **caractérisée en ce qu'**elle peut comprendre, à titre d'excipient physiologiquement compatible avec la peau, un tensioactif, un conservateur, un corps gras, un colorant, un émulsionnant, un gélifiant, un émollient, un humectant, un pigment, un antioxydant.

11. Utilisation d'un complexe tel que défini à l'une quelconque des revendications 1 à 6, pour la fabrication d'un agent cytostimulant destiné à relancer l'activité cellulaire épidermique et à limiter l'action catabolique du tissu cutané.

12. Procédé de soin cosmétique destiné à prévenir ou à réparer les dégradations cutanées liées au vieillissement, procédé mis en oeuvre en appliquant sur la peau une composition telle que définie à l'une quelconque des revendications 7 à 10.

## Patentansprüche

1. Komplex auf der Grundlage eines organischen Siliziumderivats, **dadurch gekennzeichnet, dass** das Derivat durch schwache Bindungen mit einem oder mit mehreren kalibrierten Hyaluronsäure-Fragmenten mit einem Molekulargewicht im Bereich zwischen 150 und 750 kDa, assoziiert ist, wobei das Derivat die folgende allgemeine Formel (I) aufweist:
RₓSi(OH)₄₋ₓ (I)
wobei:
R ein (C₁-C₄)-Alkyl ist,
x = 1 und 2.

2. Komplex nach Anspruch 1, **dadurch gekennzeichnet, dass** das Molekulargewicht der Fragmente im Bereich zwischen 150 und 600 kDa liegt.

3. Komplex nach einem der Ansprüche 1 und 2, **dadurch gekennzeichnet, dass** das Molekulargewicht der Fragmente im Bereich zwischen 250 und 600 kDa liegt.

4. Komplex nach einem der Ansprüche 1 bis 3, **dadurch gekennzeichnet, dass** das organische Siliziumderivat mit der Formel (I) ausgewählt ist aus dem Monomethylsilantriol oder dem Dimethylsilandiol.

5. Komplex nach Anspruch 4, **dadurch gekennzeichnet, dass** das organische Siliziumderivat das Monomethylsilantriol ist.

6. Komplex nach einem der Ansprüche 1 bis 5, **dadurch gekennzeichnet, dass** es sich um das Monomethylsilantriol handelt, assoziiert mit einem oder mit mehreren Hyaluronsäure-Fragmenten mit einem Molekulargewicht im Bereich zwischen 150 und 600 kDa oder zwischen 250 und 600 KdA.

7. Kosmetische Verbindung zur topischen Anwendung, die ausgelegt ist, um dem Haut-Abbau in Verbindung mit der Alterung vorzubeugen oder ihn zu behandeln, **dadurch gekennzeichnet, dass** sie, in Assoziation mit jedem Trägerstoff, der mit der Haut physiologisch kompatibel ist, als hauptsächlichen Wirkstoff einen Komplex nach einem der Ansprüche 1 bis 6 umfasst.

8. Zusammensetzung nach Anspruch 7, **dadurch gekennzeichnet, dass** die Menge des Komplexes mit der Formel (I) zwischen 1 und 10 Gew% mit Bezug auf das Gesamtgewicht der Zusammensetzung, vorzugsweise zwischen 2 und 5 Gew% liegt.

9. Zusammensetzung nach einem der Ansprüche 7 und 8, **dadurch gekennzeichnet, dass** sie in Form einer Creme, einer Milch, eines Gels, einer Lotion, einer Emulsion formuliert ist.

10. Zusammensetzung nach einem der Ansprüche 7 bis 9, **dadurch gekennzeichnet, dass** sie, als Trägerstoff, der mit der Haut physiologisch kompatibel ist, einen oberflächenaktiven Stoff, einen Konservierungsstoff, einen Fettkörper, einen Farbstoff, einen Emulgator, ein Geliermittel, einen Weichmacher, ein Feuchthaltemittel, ein Pigment, ein Antioxidationsmittel umfassen kann.

11. Verwendung eines Komplexes, wie in einem der Ansprüche 1 bis 6 definiert, zur Herstellung eines zytostimulierenden Mittels, das ausgelegt ist, um die epidermische Zellaktivität wiederzubeleben und die katabolische Tätigkeit des Hautgewebes zu begrenzen.

12. Verfahren zur kosmetischen Pflege, die ausgelegt ist, um dem Haut-Abbau in Verbindung mit der Alterung vorzubeugen oder ihn zu behandeln, wobei das Verfahren durchgeführt wird, indem auf die Haut eine Zusammensetzung wie in einem der Ansprüche 7 bis 10 definiert aufgebracht wird.

## Claims

1. Complex based on an organic silicon derivative **characterized in that** said derivative is combined, by weak bonds, with one or more hyaluronic acid calibrated fragments of molecular weight between 150 and 750 kDa, said derivative being of general formula (I) :
RₓSi(OH)₄₋ₓ (I)
wherein :
R is a (C₁-C₄)alkyl,
x = 1 and 2.

2. Complex according to claim 1, **characterized in that** the molecular weight of said fragments is between 150 and 600 kDa.

3. Complex according to one of claims 1 and 2, **characterized in that** the molecular weight of said fragments is between 250 and 600 kDa.

4. Complex according to any one of claims 1 to 3, **characterized in that** said organic silicon derivative of formula (I) is chosen among monomethylsilanetriol or dimethylsilanediol.

5. Complex according to claim 4, **characterized in that** the organic silicon derivative is monomethylsilanetriol.

6. Complex according to any one of claims 1 to 5, **characterized in that** monomethylsilanetriol is combined with one or more hyaluronic acid fragments of molecular weight between 150 and 600 kDa or between 250 and 600 kDa.

7. Cosmetic composition for topical application intended for preventing or repairing cutaneous damages related to aging, **characterized in that** it includes as main active ingredient a complex as defined in any one of claims 1 to 6, in combination with any excipient which is physiologically compatible with skin.

8. Composition according to claim 7, **characterized in that** the amount of complex of formula (I) is between 1 and 10% by weight in relation to the total weight of the composition, preferably between 2 and 5% by weight.

9. Composition according to any one of claims 7 and 8, **characterized in that** it is formulated under the form of cream, milk, gel, lotion, emulsion.

10. Composition according to any one of claims 7 to 9, **characterized in that** it may include as excipient which is physiologically compatible with skin, a surfactant, a preservative, a fatty substance, a dye, an emulsifier, a gelling agent, an emollient, a humectant, a pigment, an antioxidant.

11. Use of a complex as defined to any one of claims 1 to 6, for the manufacture of a cytostimulating agent intended for boosting the epidermal cell activity and for limiting the catabolic action of cutaneous tissue.

12. Cosmetic care process intended for preventing or repairing cutaneous damages related to aging, the process being achieved by applying on skin a composition as defined in any one of claims 7 to 10.
